# FASCICULE DE BREVET EUROPEEN

(11) **EP 0 542 979 B1**
(45) Date de publication et mention de la délivrance du brevet: **18.03.1998**
(21) Numéro de dépôt: 92912677.9
(22) Date de dépôt: 12.06.1992
(51) Int. Cl.: A61K 33/08, A61K 33/14, A61K 9/20, A61K 9/28

(54) **COMPOSITION THERAPEUTIQUE POUR LIBERATION PROLONGEE DE MAGNESIUM**
Magnesiumhaltiges Arzneimittel mit verlängerter Wirkstoffabgabe
THERAPEUTIC COMPOSITION FOR SUSTAINED RELEASE OF MAGNESIUM

(30) Priorité: 12.06.1991 FR 9107141
(43) Date de publication de la demande: 26.05.1993
(73) Titulaire: JOANNY, Fabienne, F-63100 Clermont-Ferrand (FR)
(72) Inventeur: JOANNY, Fabienne, F-63100 Clermont-Ferrand (FR)
(74) Mandataire: Clisci, Serge
(86) Numéro de dépôt international: FR9200534
(87) Numéro de publication internationale: WO9222305

(56) Documents cités:
- EP-A- 0 338 861
- FR-A- 2 620 334
- GB-A- 1 422 193
- US-A- 5 002 774

## Description

### DOMAINE DE L'INVENTION

La présente invention a trait au traitement des carences en magnésium. Elle concerne plus précisément une composition thérapeutique ayant une forte teneur en magnésium, le magnésium qu'elle contient étant libéré de façon lente et prolongée au niveau entéral.

### ART ANTERIEUR

On sait que Mg²⁺ est un ion essentiel pour l'activité de nombreux systèmes enzymatiques, notamment ceux intervenant dans la production d'énergie cellulaire, dans le métabolisme glucidique et dans le métabolisme des protéines.

De plus, on sait que le magnésium est essentiel pour l'intégrité et le fonctionnement des mitochondries. Le magnésium est un sédatif nerveux; il se comporte vis-à-vis du système cardiovasculaire comme un antagoniste calcique. Il est nécessaire à la croissance osseuse et à la minéralisation de l'os. Il intervient dans de nombreux processus antistress : rôle antiallergique, antianaphylactique, antiinflammatoire, diminution de la sécrétion des catécholamines.

Comme le magnésium intervient dans la quasi-totalité des fonctions physiologiques de l'organisme, une carence ou une sous-carence en magnésium entraîneront un grand nombre de troubles variés.

D'une manière générale, les apports journaliers recommandés en magnésium sont estimés à 6 mg/kg chez l'homme. Eu égard aux habitudes alimentaires, l'apport usuel quotidien en magnésium demeure dans la plupart des cas insuffisant, notamment dans les pays développés, et souvent une supplémentation en magnésium s'avère nécessaire.

On connait des compositions orales antiacides destinées au traitement des ulcères gastriques et des gastrites contenant en association un moyen couvrant et un composé de magnésium tel que MgO ou sel de magnésium. Il se trouve que les ions Mg²⁺ libérés au niveau gastrique par ces compositions ne sont guère absorbables au niveau entéral, dès lors que la vitesse d'élimination des ions Mg²⁺ par les voies naturelles est très nettement supérieure à la vitesse d'absorption desdits ions Mg²⁺ par la voie intestinale, de sorte que peu d'ions Mg²⁺ traversent la paroi intestinale pour passer dans la circulation sanguine. En conséquence, ces compositions orales anti-acides destinées au traitement des ulcères gastriques et des gastrites sont en général insuffisantes pour pallier les carences ou sous-carences en magnésium et compléter l'apport usuel quotidien en magnésium.

On sait, par ailleurs, que dans le domaine galénique on a déjà proposé des solutions techniques pour l'administration par voie orale d'un principe actif selon une dose unitaire de façon que ledit principe actif soit libéré dans l'organisme de façon lente, retardée, prolongée et/ou continue.

Ces formes galéniques, notamment décrites dans l'article de P. LOTTEAU R. Sci. Techn. Pharm., 5 (No 6), pages 335-338 (1976) et les documents de brevet GB-A-1 568 837, US-A-3 773 920, EP-A-0 330 532, FR-A-2 432 313, FR-A-2 453 641 et BE-A-882 913, ont pour but de fournir un apport continu et retardé dans le temps en principe actif, d'obtenir une action thérapeutique plus efficace et moins contraignante pour le patient dès lors que le nombre de prises du médicament est fortement réduit.

Jusqu'à présent, on n'a pas encore décrit ni proposé une solution galénique permettant l'administration unitaire de magnésium à haute dose en vue d'une libération lente et prolongée de magnésium ou d'ion magnésium dans l'organisme.

### BUT DE L'INVENTION

Un des buts de l'invention est de fournir une nouvelle solution technique permettant d'assurer un apport continu en magnésium dans l'organisme favorisant la charge cellulaire en magnésium par le biais de niveaux hématiques plus élevés pendant une durée plus longue.

Un autre but de l'invention est d'éviter une perte urinaire trop rapide du magnésium ingéré.

Un autre but de l'invention est de fournir une nouvelle solution technique au problème des carences et sous-carences en magnésium, cette solution technique étant telle que l'apport d'une teneur relativement élevée en magnésium ne perturbe pas les équilibres avec d'autres minéraux, tels que Na, K, Ca, Zn, d'une part, ni n'introduise de compétitions dans l'absorption de ces autres minéraux, tels que notamment Ca et Zn, d'autre part.

### OBJET DE L'INVENTION

Ces buts et d'autres avantages énumérés ci-après sont atteints par la nouvelle solution technique préconisée par l'invention. Cette nouvelle solution technique fait appel à une composition retard contenant une teneur en magnésium plus élevée que celle des compositions antérieurement connues, d'une part, et ayant un effet retard pour une libération lente et prolongée du magnésium au niveau entéral, d'autre part.

Ainsi, selon l'invention, on préconise une nouvelle composition thérapeutique utile vis-à-vis des carences magnésiques et destinée à assurer la libération du magnésium, qu'elle contient, de façon lente et continue sous forme de Mg²⁺ assimilable au niveau entéral afin de compléter jusqu'à au moins 6 mg/kg l'apport quotidien en magnésium chez l'homme, ladite composition étant caractérisée en ce qu'elle comporte un mélange comprenant, en association avec un excipient physiologiquement acceptable,
(A) 4 à 14 parties en poids de magnésium provenant d'une source de magnésium constituée par MgO, MgCl₂ et les hydrates de chlorure de magnésium de formule MgCl₂,nH₂O où n est un nombre entier ou fractionnaire supérieur à 0 et inférieur ou égal à 6,
(B) 6 à 13 parties en poids d'un polymère hydrophile, quand la source de Mg est MgO, ou 10 à 30 parties en poids d'une substance hydrophobe choisie parmi l'ensemble constitué par les polymères hydrophobes physiologiquement acceptables, les esters d'acide gras et leurs mélanges, quand la source de Mg est différente de MgO, et
(C) 6 à 16 parties en poids d'une charge inerte intervenant en tant que diluant solide et notamment choisie parmi l'ensemble constitué par le lactose, les phosphates de métaux alcalins et alcalino-terreux et leurs mélanges,
la teneur en Mg dans ledit mélange étant comprise entre 5 et 60 % en poids par rapport au poids dudit mélange.

Selon un autre aspect de l'invention, on préconise un procédé pour compléter l'apport usuel quotidien en magnésium tout en évitant une compétition substantielle dans l'absorption avec d'autres minéraux tels que notamment le calcium et le zinc, ledit procédé étant caractérisé en ce que l'on fait appel à une composition contenant une source de magnésium et qui
(a) a une forte teneur en magnésium, et
(b) libère lentement au niveau entéral les ions Mg²⁺ provenant de ladite source de magnésium, la vitesse d'absorption desdits ions Mg²⁺ par la paroi entérale étant supérieure à la vitesse d'élimination desdits ions Mg²⁺ par les voies naturelles.

### DESCRIPTION DETAILLEE DE L'INVENTION

Pour la mise en oeuvre de l'invention, l'on fait appel à une source de magnésium qui est choisie parmi l'ensemble constitué par MgO, MgCl₂ et MgCl₂,nH₂O où n est un nombre entier ou fractionnaire supérieur à 0 et inférieur ou égal à 6.

Les autres sources de magnésium ne conviennent pas en ce sens qu'elles conduiraient, à poids constant, à une teneur en magnésium plus faible. En effet, comme les autres sources de magnésium ont un poids moléculaire plus élevé que MgO et MgCl₂,6H₂O, il faudrait augmenter la quantité desdites autres sources pour avoir la teneur requise en magnésium thérapeutiquement efficace et on rencontrerait alors des difficultés d'administration par voie orale d'une dose unitaire d'un médicament, puisque le volume administré de produits solides serait alors trop important.

Le chlorure de magnésium, MgCl₂, étant très hygroscopique, il sera généralement utilisé sous forme hydratée. Parmi les hydrates qui conviennent selon l'invention on peut notamment citer les MgCl₂,⁹/₂H₂O et MgCl₂,6H₂O.

La source de magnésium préférée selon l'invention est constituée par MgO, MgCl₂,⁹/₂H₂O et leurs mélanges.

Jusqu'à la présente invention, on ne connaissait pas de forme retard contenant du chlorure de magnésium, eu égard au caractère hygroscopique de ce produit entraînant une instabilité de la préparation médicamenteuse. Selon l'invention, on peut maintenant administrer du chlorure de magnésium éventuellement hydraté avec un enrobage gastrorésistant en évitant l'action agressive du chlorure de magnésium au niveau de l'estomac.

Le mélange (1) comprenant les moyens (A) et (B) précités en association avec des excipients physiologiquement acceptables, peut être enrobé au moyen d'un enrobage (notamment pelliculaire) gastrorésistant. Quand le mélange (1) selon l'invention est en lui-même gastrorésistant ou est dépourvu de chlorure de magnésium éventuellement hydraté, l'enrobage gastrorésistant (2) peut être supprimé, comme illustré ci-après à l'exemple 1. Quand le mélange (1) n'est pas en lui-même gastrorésistant ou contient du chlorure de magnésium éventuellement hydraté, l'enrobage gastrorésistant (2) est nécessaire, comme illustré à l'exemple 2 ci-après.

Ainsi, la composition thérapeutique selon l'invention, utile vis-à-vis des carences magnésiques et destinée à assurer la libération entérale du magnésium, qu'elle contient, comporte
(1) un mélange comprenant, en association avec un excipient physiologiquement acceptable,
   (A) 4 à 14 parties en poids de magnésium provenant d'une source de magnésium constituée par MgO, MgCl₂ et les hydrates de chlorure de magnésium de formule MgCl₂,nH₂O où n est un nombre entier ou fractionnaire supérieur à 0 et inférieur ou égal à 6,
   (B) 6 à 13 parties en poids d'un polymère hydrophile, quand la source de Mg est MgO, ou 10 à 30 parties en poids d'une substance hydrophobe choisie parmi l'ensemble constitué par les polymères hydrophobes physiologiquement acceptables, les esters d'acide gras et leurs mélanges, quand la source de Mg est différente de MgO, et
   (C) 6 à 16 parties en poids d'une charge inerte intervenant en tant que diluant solide et notamment choisie parmi l'ensemble constitué par le lactose, les phosphates de métaux alcalins et alcalino-terreux et leurs mélanges,
   la teneur en Mg dans ledit mélange étant comprise entre 5 et 60 % en poids par rapport au poids dudit mélange; et, le cas échéant,
(2) un enrobage gastrorésistant..

Selon l'invention, quand la source de magnésium est MgO, le mélange (1) comprend avantageusement en tant que moyen (B) un polymère hydrophile choisi parmi l'ensemble constitué par les polymères cellulosiques, notamment la carboxyméthylcellulose, l'hydroxypropylcellulose, l'hydroxypropylméthylcellulose et leurs mélanges.

Quand la source de magnésium est MgCl₂ (et mieux un hydrate de MgCl₂ contenant au plus 6 molécules d'eau pour une molécule de chlorure de magnésium), le moyen (B) du mélange (1) comprend avantageusement un polymère hydrophobe, notamment le poly(chlorure de vinyle) ou l'éthylcellulose, et un composant ester d'acide gras constitué d'un ou plusieurs esters. De façon avantageuse, ledit composant ester d'acide gras comprend au moins un reste d'acide gras ayant notamment de 8 à 24 atomes de carbone et au moins un reste de polyol, tel que notamment un reste de glycérol et/ou de polyéthylèneglycol; ledit composant ester d'acide gras peut être un mélange de ces esters.

Le mélange (1) peut contenir d'autres ingrédients. Parmi ces ingrédients, on recommande l'utilisation d'une charge inerte telle que le lactose et les phosphates de métaux alcalins et alcalino-terreux, à savoir comme indiqué ci-dessus :
(C) 6 à 16 parties en poids d'une charge inerte intervenant en tant que diluant solide et notamment choisie parmi l'ensemble constitué par le lactose, les phosphates de métaux alcalins et alcalino-terreux et leurs mélanges.

En pratique, ladite charge inerte sera avantageusement le lactose quand la source de magnésium est MgO, d'une part, et le lactose, le phosphate dicalcique ou un de leurs mélanges quand la source de magnésium est MgCl₂ hydraté, notamment MgCl₂,⁹/₂H₂O, d'autre part.

Parmi les autres ingrédients pouvant être incorporés dans le mélange (1), on peut notamment citer (i) la silice colloïdale qui intervient en tant que charge, d'une part, et liant, d'autre part, (ii) la polyvinylpyrrolidone qui intervient en tant que liant, (iii) un moyen lubrifiant et (iv) d'autres ingrédients actifs tels que par exemple KCl, la vitamine B6 et, le cas échéant, la vitamine C ou la vitamine A.

L'enrobage (2) sera avantageusement un enrobage pelliculaire constitué par un mélange d'acétophtalate de cellulose et de polyéthylèneglycol.

Selon le meilleur mode de mise en oeuvre de l'invention, la composition thérapeutique contenant une source de magnésium sera présentée sous la forme d'un comprimé, le mélange (1) ayant été comprimé selon une méthode galénique usuelle puis éventuellement revêtu de l'enrobage gastrorésistant (2).

Selon ce meilleur mode, la composition selon l'invention sera adaptée pour une prise unitaire quotidienne et contiendra avantageusement de 40 à 140 mg (et mieux d'environ 50 à environ 120 mg) de magnésium selon la source de magnésium utilisée.

La composition thérapeutique selon l'invention assure donc une libération lente et continue du magnésium au niveau entéral et évite, eu égard à la cinétique de libération des ions Mg²⁺ au niveau de l'intestin une compétition dans l'absorption avec d'autres minéraux tels que le calcium et le zinc. Elle permet, par ailleurs, d'avoir une source continue en magnésium sans pics plasmatiques suivis d'une diminution rapide de la magnésémie, à la différence de l'administration du magnésium par voie injectable suivant laquelle au moins la moitié du Mg injecté par voie intraveineuse est éliminée en environ une heure par les voies naturelles.

La source de magnésium constituée par MgO est celle qui permet d'apporter le maximum de magnésium assimilable dans un volume minimal.

D'autres avantages et caractéristiques de l'invention seront mieux compris à la lecture d'exemples de réalisation nullement limitatifs mais donnés à titre d'illustration.

### EXEMPLE 1

### Comprimés retard contenant MgO

On a préparé des comprimés contenant 200 mg d'oxyde de magnésium par unité (soit un apport de 120 mg en Mg par unité de prise), ces comprimés ayant la formulation I suivante en poids.

| **Formulation I** | |
|---|---|
| MgO | 32 % |
| KCl | 24,5 % |
| Chlorhydrate de pyridoxine | 8 % |
| Lactose | 14,85 % |
| Polyvinylpyrrolidone ("Polyvidone K 29-32") | 2,4 % |
| Carboxyméthylcellulose sodique | 15,7 % |
| Silice colloidale | 0,15 % |
| Béhénate de glycérol | 2,4 % |

On prépare des granulés selon la méthode de granulation dite par voie humide en utilisant un moyen diluant solide tel que le lactose, un moyen liant tel que la polyvinylpyrrolidone et un liquide de mouillage tel que l'éthanol dilué (avec de l'eau) à 60°. Les modalités opératoires sont les suivantes.
(a) Les principes actifs (MgO, KCl et le chlorhydrate de pyridoxine) sont préalablement tamisés sur un tamis ayant une ouverture de maille de 0,400 mm, puis mélangés dans un mélangeur de poudre (du type mélangeur cubique) pendant 20 minutes.
(b) Le mélange ainsi obtenu est introduit dans un mélangeur malaxeur puis humidifié avec le liquide de mouillage.
(c) La masse obtenue au stade (b) est ensuite granulée au moyen d'un granulateur pourvu d'une grille ayant une ouverture de maille d'environ 2 mm.
(d) Les granulés humides ainsi obtenus sont alors séchés à 50-70°C, notamment dans une étuve ventilée pendant environ 4 h à 60°C ou dans un séchoir par fluidisation pendant 0,5 h à 60°C.
(e) Les granulés séchés ainsi obtenus sont calibrés dans un granulateur équipé d'une grille ayant une ouverture de maille d'environ 1 mm ou 1,2 mm.
(f) Les granulés ainsi obtenus sont introduits dans un mélangeur à poudre (notamment du type cubique) et on ajoute le polymère cellulosique (carboxyméthylcellulose sodique) et la silice colloidale. Le mélange des produits ainsi introduits est effectué pendant 20 minutes. On ajoute alors le béhénate de glycérol (en tant que moyen plastifiant) et le mélange des ingrédients est encore poursuivi pendant 20 minutes.
(g) Le mélange résultant est comprimé, notamment au moyen de pastilleuses munies de poinçons concaves ayant un diamètre de 12 mm et un rayon de courbure de 11 mm.

Le comprimé ainsi obtenu convient parfaitement pour la libération du magnésium au niveau entéral.

### EXEMPLE 2

### Comprimés retard contenant MgCl₂,⁹/₂H₂O

On a préparé des comprimés contenant 363 mg de MgCl₂,⁹/₂H₂O par unité (soit un apport de 50 mg en Mg par unité de prise), ces comprimés ayant la formulation II suivante en poids.

| **Formulation II** | |
|---|---|
| MgCl₂,⁹/₂H₂O | 44,5 % |
| Chlorhydrate de pyridoxine | 3 % |
| Lactose | 14,78 % |
| Poly(chlorure de vinyle) | 18,4 % |
| Silice colloidale anhydre | 0,92 % |
| Ester d'acide gras | 18,4 % |

On prépare les comprimés selon une méthode galénique classique en utilisant un composé plastique du type poly(chlorure de vinyle) et un composant ester d'acide gras (un ou plusieurs esters d'acide gras avec le glycérol ou le polyéthylèneglycol) et en opérant dans un local d'humidité relative normale (HR d'au plus 50-60 %). Les modalités opératoires sont les suivantes.
(a) Après tamisage des ingrédients actifs (MgCl₂,⁹/₂H₂O et chlorhydrate de pyridoxine) sur un tamis ayant une ouverture de maille d'environ 0,400 mm, on incorpore le lactose, le poly(chlorure de vinyle), la silice colloidale et une fraction du composant ester d'acide gras dans un mélangeur à poudre (notamment du type cubique). Le mélange de ces ingrédients est effectué pendant 0,5 h.
(b) Le mélange résultant est comprimé notamment au moyen de pastilleuses pourvues de poinçons ayant un diamètre de 22 mm.
(c) Les comprimés ainsi obtenus sont broyés puis calibrés sur un granulateur muni d'une grille ayant une ouverture de maille de 1 ou 1,2 mm.
(d) Dans un mélangeur de type cubique, les granulés ainsi obtenus sont incorporés avec le reste du composant ester d'acide gras. On mélange le produit résultant pendant 0,5 h.
(e) Le mélange ainsi obtenu est soumis à une compression au moyen de pastilleuses équipées de poinçons biconcaves d'un diamètre de 12 mm et d'un rayon de courbure de 11 mm.
(f) Les comprimés résultants sont ensuite pelliculés avec une solution d'un produit filmogène gastrorésistant (notamment du type acétophtalate de cellulose) et d'un moyen plastifiant (polyéthylèneglycol de poids moléculaire moyen 400), à raison de 1,35 % en poids de plastifiant par rapport au poids du produit filmogène, dans un mélange chloroforme-éthanol (d'environ 1/1 v/v). Cette solution est répartie sur les comprimés au moyen d'une turbine à dragéifier ou dans un dispositif de revêtement pelliculaire du type ACELA COTA ou GLATT, de façon à déposer 15 mg en poids sec de composition filmogène par comprimé.
(g) Les comprimés ainsi revêtus sont placés dans des récipients étanches contenant des sachets de moyen déshydratant en attendant le conditionnement définitif.

### EXEMPLE 3

### Mesure de la cinétique de la libération du magnésium

La technique, qui a été suivie pour évaluer la cinétique de la libération du magnésium ***in vitro***, est celle préconisée par la Pharmacopée Française. Elle met en oeuvre une méthode utilisant une palette tournante à 60 tours/minute dans un milieu tamponné à pH 1,3 pour les comprimés d'oxyde de magnésium et à pH 6,8 pour les comprimés de chlorure de magnésium éventuellement hydraté.

Les résultats, qui ont été obtenus, sont consignés dans les tableaux 1 et 2 ci-après, où est donnée la libération du magnésium en pourcentage par rapport à la quantité totale de magnésium contenue dans chaque comprimé.

Le tableau 1 fournit les résultats pour le comprimé de formulation I ayant pour principe actif l'oxyde de magnésium selon l'exemple 1.

Le tableau 2 fournit les résultats pour le comprimé de formulation II ayant pour principe actif le chlorure de magnésium hydraté avec 4,5 molécules d'eau MgCl₂,⁹/₂H₂O selon l'exemple 2.

Les valeurs données dans lesdits tableaux 1 et 2 mettent en évidence l'intérêt de la composition selon l'invention pour l'administration du magnésium.

### EXEMPLE 4

En procédant comme indiqué à l'exemple 2, mais en remplaçant le lactose (14,78 % en poids) par une quantité identique (14,78 % en poids) de phosphate dicalcique, on obtient un comprimé enrobé présentant une cinétique de libération du magnésium similaire à celle de l'exemple 2.

### EXEMPLE 5

En procédant comme indiqué à l'exemple 2, mais en remplaçant le lactose (14,78 % en poids) par une quantité identique (14,78 % en poids) d'un mélange de lactose (6,53 % en poids) et de phosphate dicalcique (8,20 % en poids), on obtient un comprimé enrobé présentant une cinétique de libération du magnésium similaire à celle de l'exemple 2.

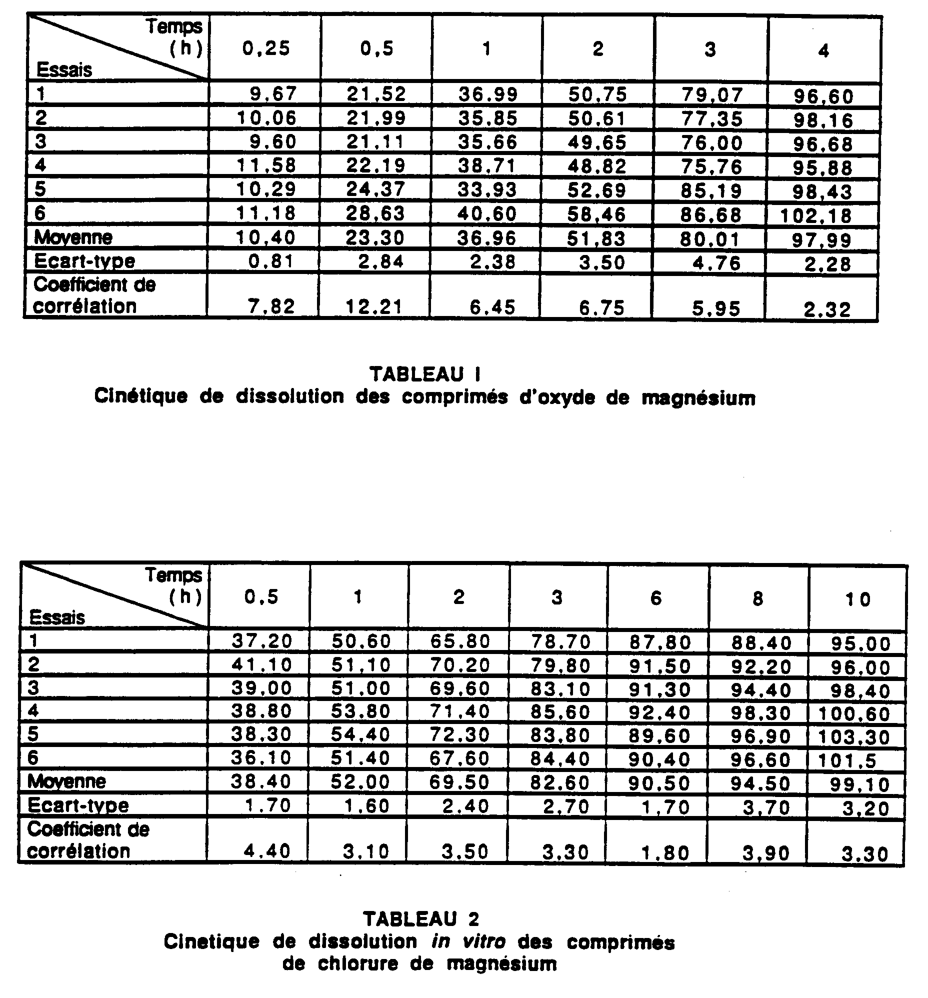

## Revendications (Revendications pour l'(les) Etat(s) contractant(s) suivant(s): AT, BE, CH, DE, DK, FR, GB, IT, LI, MC, NL, SE)

1. Composition thérapeutique utile vis-à-vis des carences magnésiques et destinée à assurer la libération du magnésium, qu'elle contient, de façon lente et continue sous forme de Mg²⁺ assimilable au niveau entéral afin de compléter jusqu'à au moins 6 mg/kg l'apport quotidien en magnésium chez l'homme, ladite composition étant caractérisée en ce qu'elle comporte un mélange comprenant, en association avec un excipient physiologiquement acceptable,
(A) 4 à 14 parties en poids de magnésium provenant d'une source de magnésium constituée par MgO, MgCl₂ et les hydrates de chlorure de magnésium de formule MgCl₂, nH₂O où n est un nombre entier ou fractionnaire supérieur à 0 et inférieur ou égal à 6,
(B) 6 à 13 parties en poids d'un polymère hydrophile, quand la source de Mg est MgO, ou 10 à 30 parties en poids d'une substance hydrophobe choisie parmi l'ensemble constitué par les polymères hydrophobes physiologiquement acceptables, les esters d'acide gras et leurs mélanges, quand la source de Mg est différente de MgO, et
(C) 6 à 16 parties en poids d'une charge inerte intervenant en tant que diluant solide et notamment choisie parmi l'ensemble constitué par le lactose, les phosphates de métaux alcalins et alcalino-terreux et leurs mélanges,
la teneur en Mg dans ledit mélange étant comprise entre 5 et 60 % en poids par rapport au poids dudit mélange.

2. Composition suivant la revendication 1, caractérisée en ce que la source de magnésium est MgO.

3. Composition suivant la revendication 1, caractérisée en ce que la source de magnésium est MgCl₂,⁹/₂H₂O.

4. Composition suivant la revendication 1, caractérisée en ce que le moyen (B) est un polymère hydrophile choisi parmi l'ensemble constitué par les polymères cellulosiques, notamment la carboxyméthylcellulose, l'hydroxypropylcellulose, l'hydroxypropylméthylcellulose et leurs mélanges.

5. Composition suivant la revendication 1, caractérisée en ce que le moyen (C) est le lactose, le phosphate dicalcique ou un de leurs mélanges.

6. Composition suivant la revendication 1, caractérisée en ce que ledit mélange (1) comprenant la source de magnésium (A), le moyen (B), les excipients et le moyen (C) est revêtu d'un enrobage gastrorésistant (2).

7. Composition suivant la revendication 6, caractérisée en ce que ledit enrobage gastrorésistant (2) est constitué d'un mélange d'acétophtalate de cellulose et de polyéthylèneglycol.

8. Composition suivant l'une quelconque des revendications 1 à 7, caractérisée en ce qu'elle est présentée sous une forme unitaire pour administration d'une seule dose quotidienne, contient une source de magnésium correspondant à une quantité de magnésium de 40 à 140 mg et mieux de 50 à 120 mg, et est dépourvue d'une source de potassium.

## Revendications (Revendications pour l'(les) Etat(s) contractant(s) suivant(s): ES, GR)

1. Procédé pour la préparation d'une composition thérapeutique utile vis-à-vis des carences magnésiques et destinée à assurer la libération du magnésium, qu'elle contient, de façon lente et continue sous forme de Mg²⁺ assimilable au niveau entéral afin de compléter jusqu'à au moins 6 mg/kg l'apport quotidien en magnésium chez l'homme, ledit procédé étant caractérisé en ce qu'il met en oeuvre un mélange comprenant, en association avec un excipient physiologiquement acceptable,
(A) 4 à 14 parties en poids de magnésium provenant d'une source de magnésium constituée par MgO, MgCl₂ et les hydrates de chlorure de magnésium de formule MgCl₂, nH₂O où n est un nombre entier ou fractionnaire supérieur à 0 et inférieur ou égal à 6,
(B) 6 à 13 parties en poids d'un polymère hydrophile, quand la source de Mg est MgO, ou 10 à 30 parties en poids d'une substance hydrophobe choisie parmi l'ensemble constitué par les polymères hydrophobes physiologiquement acceptables, les esters d'acide gras et leurs mélanges, quand la source de Mg est différente de MgO, et
(C) 6 à 16 parties en poids d'une charge inerte intervenant en tant que diluant solide et notamment choisie parmi l'ensemble constitué par le lactose, les phosphates de métaux alcalins et alcalino-terreux et leurs mélanges,
la teneur en Mg dans ledit mélange étant comprise entre 5 et 60 % en poids par rapport au poids dudit mélange.

2. Procédé suivant la revendication 1, caractérisé en ce que la source de magnésium est MgO.

3. Procédé suivant la revendication 1, caractérisé en ce que la source de magnésium est MgCl₂,⁹/₂H₂O.

4. Procédé suivant la revendication 1, caractérisé en ce que le moyen (B) est un polymère hydrophile choisi parmi l'ensemble constitué par les polymères cellulosiques, notamment la carboxyméthylcellulose, l'hydroxypropylcellulose, l'hydroxypropylméthylcellulose et leurs mélanges.

5. Procédé suivant la revendication 1, caractérisé en ce que le moyen (C) est le lactose, le phosphate dicalcique ou un de leurs mélanges.

6. Procédé suivant la revendication 1, caractérisé en ce que ledit mélange (1) comprenant la source de magnésium (A), le moyen (B), les excipients et le moyen (C) est revêtu d'un enrobage gastrorésistant (2).

7. Procédé suivant la revendication 6, caractérisé en ce que ledit enrobage gastrorésistant (2) est constitué d'un mélange d'acétophtalate de cellulose et de polyéthylèneglycol.

8. Procédé suivant l'une quelconque des revendications 1 à 7, pour la préparation d'une composition qui se présente sous une forme unitaire pour administration d'une seule dose quotidienne, contient une source de magnésium correspondant à une quantité de magnésium de 40 à 140 mg et mieux de 50 à 120 mg, et est dépourvue d'une source de potassium.

## Claims (Claims for the following Contracting State(s): AT, BE, CH, DE, DK, FR, GB, IT, LI, MC, NL, SE)

1. A therapeutic or nutritional composition useful vis-a-vis magnesium deficiencies and intended to ensure that the magnesium which it contains is released slowly and continuously in the form of assimilable Mg²⁺ in the intestine, so as to make up the daily intake of magnesium in man to at least 6 mg/kg, said composition being characterized in that it contains a mixture comprising, in association with a physiologically acceptable excipient,
(A) 4 to 14 parts by weight of magnesium derived from a magnesium source consisting of MgO, MgCl₂ and magnesium chloride hydrates of the formula MgCl₂.nH₂O, where n is an integer or fraction greater than 0 and less than or equal to 6,
(B) 6 to 13 parts by weight of a hydrophilic polymer, when the Mg source is MgO, or 10 to 30 parts by weight of a hydrophobic substance selected from the group consisting of physiologically acceptable hydrophobic polymers, fatty acid esters and mixtures thereof, when the Mg source is other than MgO, and
(C) 6 to 16 parts by weight of an inert filler present as a solid diluent and selected especially from the group consisting of lactose, alkali metal and alkaline earth metal phosphates and mixtures thereof,
the Mg content of said mixture being between 5 and 60 % by weight based on the weight of said mixture.

2. A composition according to claim 1, wherein the magnesium source is MgO.

3. A composition according to claim 1, wherein the magnesium source is MgCl₂.9/2H₂O.

4. A composition according to claim 1, wherein substance (B) is a hydrophilic polymer selected from the group consisting of cellulosic polymers, especially carboxymethyl cellulose, hydroxypropyl cellulose, hydroxypropyl methyl cellulose and mixtures thereof.

5. A composition according to claim 1, wherein substance (C) is lactose, dicalcium phosphate or a mixture thereof.

6. A composition according to claim 1, wherein said mixture (1), comprising the magnesium source (A), the substance (B), the excipients and, if necessary, the substance (C), is coated with a coating (2) resistant to gastric juices.

7. A composition according to claim 6, wherein said coating (2) resistant to gastric juices consists of a mixture of cellulose acetophthalate and polyethylene glycol.

8. A composition according to any one of claims 1 to 7 which is presented in a unit form for the administration of a single daily dose, contains a magnesium source corresponding to an amount of magnesium of 40 to 140 mg and preferably 50 to 120 mg, and is free of any potassium source.

## Claims (Claims for the following Contracting State(s): ES, GR)

1. A method for preparing a therapeutic or nutritional composition useful vis-a-vis magnesium deficiencies and intended to ensure that the magnesium which it contains is released slowly and continuously in the form of assimilable Mg²⁺ in the intestine, so as to make up the daily intake of magnesium in man to at least 6 mg/kg, said method comprising providing a mixture comprising, in association with a physiologically acceptable excipient,
(A) 4 to 14 parts by weight of magnesium derived from a magnesium source consisting of MgO, MgCl₂ and magnesium chloride hydrates of the formula MgCl₂, nH₂O, where n is an integer or fraction greater than 0 and less than or equal to 6,
(B) 6 to 13 parts by weight of a hydrophilic polymer, when the Mg source is MgO, or 10 to 30 parts by weight of a hydrophobic substance selected from the group consisting of physiologically acceptable hydrophobic polymers, fatty acid esters and mixtures thereof, when the Mg source is other than MgO, and
(C) 6 to 16 parts by weight of an inert filler present as a solid diluent and selected especially from the group consisting of lactose, alkali metal and alkaline earth metal phosphates and mixtures thereof,
the Mg content of said mixture being between 5 and 60 % by weight based on the weight of said mixture.

2. A method according to claim 1, wherein the magnesium source is MgO.

3. A method according to claim 1, wherein the magnesium source is MgCl₂.9/2H₂O.

4. A method according to claim 1, wherein substance (B) is a hydrophilic polymer selected from the group consisting of cellulosic polymers, especially carboxymethyl cellulose, hydroxypropyl cellulose, hydroxypropyl methyl cellulose and mixtures thereof.

5. A method according to claim 1, wherein substance (C) is lactose, dicalcium phosphate or a mixture thereof.

6. A method according to claim 1, wherein said mixture (1), comprising the magnesium source (A), the substance (B), the excipients and, if necessary, the substance (C), is coated with a coating (2) resistant to gastric juices.

7. A method according to claim 6, wherein said coating (2) resistant to gastric juices consists of a mixture of cellulose acetophthalate and polyethylene glycol.

8. A method according to any one of claims 1 to 7 for the preparation of a composition, which is presented in a unit form for the administration of a single daily dose, contains a magnesium source corresponding to an amount of magnesium of 40 to 140 mg and preferably 50 to 120 mg, and is free of any potassium source.

## Patentansprüche (Patentansprüche für folgende(n) Vertragsstaat(en): AT, BE, CH, DE, DK, FR, GB, IT, LI, MC, NL, SE)

1. Therapeutische Zusammensetzung oder Zusammensetzung für eine ausgewogene Ernährung für die Anwendung bei Magnesiummangelerscheinungen, und dafür vorgesehen die Freisetzung des in der Zusammensetzung enthaltenden Magnesiums zu gewährleisten, das langsam und kontinuierlich im Darmbereich als Mg²⁺ aufnahmefähig ist, um bis hin zu wenigstens 6 mg/kg die tägliche Zufuhr von Magnesium beim Menschen zu vervollständigen,
**dadurch gekennzeichnet**, daß
die Zusammensetzung eine Verbindung enthält, die zusammen mit einem physiologisch verträglichem Bindemittel folgendes enthält:
(A): 4 bis 14 Gew.-Teile Magnesium, das aus einer Magnesiumquelle aus der Gruppe bestehend aus MgO, MgCl₂ und Magnesiumchloridhydraten der Formel MgCl₂,nH₂O stammt, wobei n eine ganze Zahl oder ein Bruch größer 0 und kleiner oder gleich 6 ist,
(B): 6 bis 13 Gew.-Teile eines hydrophilen Polymers, wenn die Magnesium-Quelle MgO ist, oder 10 bis 30 Gew.-Teile einer hydrophoben Substanz ausgewählt aus der Gruppe bestehend aus physiologisch verträglichen hydrophoben Polymeren, Fettsäureestern und deren Verbindungen, wenn die Magnesium-Quelle nicht MgO ist, und
(C): 6 bis 16 Gew.-Teile eines intervenierenden inerten Anteils als dauerhafter Verdünner und insbesondere ausgewählt aus der Gruppe bestehend aus Lactose, Alkaliphosphaten und Erdalkaliphosphaten und deren Verbindungen,
und der Mg-Gehalt in der Verbindung bezogen auf das Gewicht der Verbindung zwischen 5 bis 60 Gew.-% beträgt.

2. Zusammensetzung nach Anspruch 1, dadurch gekennzeichnet, daß die Magnesiumquelle MgO ist.

3. Zusammensetzung nach Anspruch 1, dadurch gekennzeichnet, daß die Magnesiumquelle MgCl₂, 9/2 H₂O ist.

4. Zusammensetzung nach Anspruch 1, dadurch gekennzeichnet, daß das Mittel (B) ein hydrophiles Polymer ist, das aus der Gruppe bestehend aus Cellulosepolymeren, nämlich Carboxymethylcellulose, Hydroxypropylcellulose, Hydroxypropylmethylcellulose und deren Verbindungen ausgewählt ist.

5. Zusammensetzung nach Anspruch 1, dadurch gekennzeichnet, daß das Mittel (C) Lactose, Dicalciumphosphat oder eine deren Verbindungen ist.

6. Zusammensetzung nach Anspruch 1, dadurch gekennzeichnet, daß die Verbindung (1) aus der Magnesiumquelle (A), dem Mittel (B), den Bindemitteln und dem Mittel (C) besteht und mit einem magenresistenten Mantel (2) überzogen ist.

7. Zusammensetzung nach Anspruch 6, dadurch gekennzeichnet, daß der magenresistente Mantel (2) aus einer Verbindung von Cellulose-Acetophthalat und Polyethylenglykol besteht.

8. Zusammensetzung nach einem der Ansprüche 1 bis 7, dadurch gekennzeichnet, daß die Zusammensetzung in einer einheitlichen Form vorliegt, so daß eine einzige tägliche Dosis verabreicht werden kann, die eine Magnesiumquelle von 40 bis 140 mg und vorzugsweise von 50 bis 120 mg darstellt und ohne einer Kaliumquelle auskommt.

## Patentansprüche (Patentansprüche für folgende(n) Vertragsstaat(en): ES, GR)

1. Verfahren zur Herstellung einer therapeutischen Zusammensetzung oder Zusammensetzung für eine ausgewogene Ernährung für die Anwendung bei Magnesiummangelerscheinungen, und dafür vorgesehen die Freisetzung von Magnesium zu gewährleisten, das langsam und kontinuierlich im Darmbereich unter der Bildung von Mg²⁺ aufnahmefähig ist, um bis hin zu wenigstens 6 mg/kg die tägliche Zufuhr von Magnesium beim Menschen zu vervollständigen,
**dadurch gekennzeichnet**, daß
mit dem Verfahren die Zusammensetzung eine Verbindung enthält, die zusammen mit einem physiologisch verträglichem Bindemittel aus folgenden Mitteln bereitgestellt wird:
(A): 4 bis 14 Gew.-Teile Magnesium, das aus einer Magnesiumquelle aus der Gruppe bestehend aus MgO, MgCl₂ und den Magnesiumchloridhydraten der Formel MgCl₂,nH₂O stammt, wobei n eine ganze Zahl oder ein Bruch größer 0 und kleiner oder gleich 6 ist,
(B): 6 bis 13 Gew.-Teile eines hydrophilen Polymers, wenn die Magnesium-Quelle MgO ist, oder 10 bis 30 Gew.-Teile einer hydrophoben Substanz ausgewählt aus der Gruppe bestehend aus physiologisch verträglichen hydrophoben Polymeren, Fettsäureestern und deren Verbindungen, wenn die Magnesium-Quelle nicht MgO ist, und
(C): 6 bis 16 Gew.-Teile eines intervenierenden inerten Anteils als dauerhafter Verdünner und insbesondere ausgewählt aus der Gruppe bestehend aus Lactose, Alkaliphosphaten und Erdalkaliphosphaten und deren Verbindungen,
und der Mg-Gehalt in der Verbindung bezogen auf das Gewicht der Verbindung zwischen 5 bis 60 Gew.-% beträgt.

2. Verfahren nach Anspruch 1, dadurch gekennzeichnet, daß die Magnesiumquelle MgO ist.

3. Verfahren nach Anspruch 1, dadurch gekennzeichnet, daß die Magnesiumquelle MgCl₂, 9/2 H₂O ist.

4. Verfahren nach Anspruch 1, dadurch gekennzeichnet, daß das Mittel (B) ein hydrophiles Polymer ist, das aus der Gruppe bestehend aus Cellulosepolymeren, nämlich Carboxymethylcellulose, Hydroxypropylcellulose, Hydroxypropylmethylcellulose und deren Verbindungen ausgewählt ist.

5. Verfahren nach Anspruch 1, dadurch gekennzeichnet, daß das Mittel (C) Lactose, Dicalciumphosphat oder eine deren Verbindungen ist.

6. Verfahren nach Anspruch 1, dadurch gekennzeichnet, daß die Verbindung (1) aus der Magnesiumquelle (A), dem Mittel (B), den Bindemitteln und dem Mittel (C) besteht und mit einem magenresistenten Mantel (2) überzogen ist.

7. Verfahren nach Anspruch 6, dadurch gekennzeichnet, daß der magenresistente Mantel (2) aus einer Verbindung von Cellulose-Acetophthalat und Polyethylenglykol besteht.

8. Verfahren nach einem der Ansprüche 1 bis 7, dadurch gekennzeichnet, daß die Zusammensetzung in einer einheitlichen Form vorliegt, so daß eine einzige tägliche Dosis verabreicht werden kann, die eine Magnesiumquelle von 40 bis 140 mg und vorzugsweise von 50 bis 120 mg darstellt und ohne eine Kaliumquelle auskommt.
